# EUROPÄISCHE PATENTSCHRIFT

(11) **EP 3 092 092 B1**
(45) Veröffentlichungstag und Bekanntmachung des Hinweises auf die Patenterteilung: **28.04.2021**
(21) Anmeldenummer: 14830788.7
(22) Anmeldetag: 19.12.2014
(51) Int. Cl.: B22C 1/00, B22C 1/16

(54) **VERFAHREN ZUR HERSTELLUNG VON FORMEN UND KERNEN FÜR DEN METALLGUSS UNTER VERWENDUNG EINER CARBONYLVERBINDUNG SOWIE NACH DIESEM VERFAHREN HERGESTELLTE FORMEN UND KERNE**
METHOD FOR PRODUCING MOULDS AND CORES FOR METAL CASTING, USING A CARBONYL COMPOUND, AND MOULDS AND CORES PRODUCED ACCORDING TO SAID METHOD
PROCÉDÉ DE RÉALISATION DE MOULES ET DE NOYAUX POUR LA COULÉE DE MÉTAUX PAR UTILISATION D'UN COMPOSÉ DE CARBONYLE ET MOULES ET NOYAUX RÉALISÉS GRÂCE À CE PROCÉDÉ

(30) Priorität: 19.12.2013 DE 102013114581
(43) Veröffentlichungstag der Anmeldung: 16.11.2016
(73) Patentinhaber: ASK Chemicals GmbH, 40721 Hilden (DE)
(72) Erfinder: DETERS, Heinz, 40239 Düsseldorf (DE); LINCKE, Hannes, 51063 Köln (DE); RESCH, Ronja, 42553 Velbert (DE); SCHMIDT, Oliver, 40764 Langenfeld (DE)
(74) Vertreter: Müller Schupfner & Partner Patent- und Rechtsanwaltspartnerschaft mbB
(86) Internationale Anmeldenummer: PCT/DE2014/000640
(87) Internationale Veröffentlichungsnummer: WO 2015/090269

(56) Entgegenhaltungen:
- WO-A1-2013/159762
- AT-B- 341 125
- CA-A- 1 191 016
- CN-A- 1 212 913
- CN-A- 101 947 637
- DE-A1- 3 600 956
- DE-A1-102012 104 934
- JIN G ET AL: "The research on core making technology in sodium silicate cold box", PROCEEDINGS OF 69TH WORLD FOUNDRY CONGRESS, WFC 2010 - HANGZHOU, CHINA, 16-20 OCTOBER 2010,, 16. Oktober 2010 (2010-10-16), Seite 1000/823, XP009183613, ISBN: 978-1-62276-286-6

## Beschreibung

Die Erfindung betrifft ein Verfahren zur Herstellung von Gießformen und Kernen, bei dem eine Formstoffmischung aus mindestens einem Feuerfeststoff und Wasserglas als anorganischem Bindemittel mit Hilfe einer in die Gasphase gebrachten oder mit der Gasphase mitgeführten Carbonylverbindung ausgehärtet wird. Weiterhin betrifft die Erfindung eine entsprechende Formstoffmischung, ein Mehrkomponentensystem und die nach dem Verfahren hergestellten Formen und Kerne.

### Stand der Technik

Für die Herstellung von Formen und Kernen verwendet man i.A. einen feuerfesten Formgrundstoff, z.B. Quarzsand, und ein geeignetes Bindemittel. Der feuerfeste Formgrundstoff liegt dabei bevorzugt in einer rieselfähigen Form vor, so dass das Gemisch aus Formgrundstoff und Bindemittel, die sogenannte Formstoffmischung, in eine geeignete Hohlform eingefüllt, dort verdichtet und ausgehärtet werden kann. Durch das Bindemittel wird ein fester Zusammenhalt zwischen den Partikeln des Formgrundstoffs erzeugt, so dass die Formen und Kerne die erforderliche mechanische Stabilität erhalten.

Formen bilden beim Gießen die äußere Wandung für das Gussstück, Kerne werden dann eingesetzt, wenn Hohlräume innerhalb des Gussstückes erforderlich sind. Dabei ist es nicht zwingend notwendig, dass Formen und Kerne aus demselben Material bestehen. So erfolgt z.B. beim Kokillenguss die äußere Formgebung der Gussstücke mit Hilfe metallischer Dauerformen. Auch ist eine Kombination von Formen und Kernen, die nach unterschiedlichen Verfahren produziert wurden, möglich. Was nachfolgend anhand von Kernen erläutert wird, gilt analog auch für nach demselben Verfahren hergestellte Formen (Gießformen) und umgekehrt.

Zur Herstellung von Kernen können sowohl organische als auch anorganische Bindemittel eingesetzt werden, deren Aushärtung jeweils durch kalte oder heiße Verfahren erfolgen kann. Als kalte Verfahren bezeichnet man dabei Verfahren, welche im Wesentlichen bei Raumtemperatur (25 °C) ohne Erhitzen des zur Kernherstellung verwendeten Formwerkzeugs durchgeführt werden.

Die Aushärtung erfolgt dabei meist durch eine chemische Reaktion, die beispielsweise dadurch ausgelöst wird, dass ein Gas durch die zu härtende Formstoffmischung geleitet wird. Bei heißen Verfahren wird die Formstoffmischung nach der Formgebung durch das erwärmte Formwerkzeug auf eine ausreichend hohe Temperatur erhitzt, um beispielsweise das im Bindemittel enthaltene Lösungsmittel auszutreiben und/oder um eine chemische Reaktion zu initiieren, durch welche das Bindemittel z.B. durch Vernetzen ausgehärtet wird.

Im Bereich kalthärtender Kernherstellungsverfahren fand noch bis in die 50er und 60er Jahre des 20. Jahrhunderts das Wasserglas-CO₂-Verfahren eine breite Anwendung. Bei diesem Verfahren, wie es z.B. aus der GB 654817 bekannt ist, wird CO₂ zur Aushärtung des auf Wasserglas basierenden anorganischen Bindemittels eingesetzt. Einer der Schwachpunkte dieses Verfahrens ist, dass die daraus hergestellten Kerne relativ geringe Festigkeiten aufweisen, insbesondere unmittelbar nach ihrer Herstellung. Des Weiteren erlaubt das Wasserglas-CO₂-Verfahren nur geringe bis mittlere Fertigungsgeschwindigkeiten.

Gute Festigkeiten bereits nach kurzen Härtungszeiten sind jedoch notwendig, um die immer komplizierter werdenden dünnwandigen Gießformen, wie sie heute immer häufiger gefordert werden, sicher handhaben zu können und um gleichzeitig eine hohe Produktivität zu gewährleisten. Es ist daher nicht verwunderlich, dass das Wasserglas-CO₂-Verfahren mit dem Aufkommen von Prozessen basierend auf organischen Bindemitteln, insbesondere dem sog. Ashland-Polyurethan-ColdBox-Verfahren, rapide an Bedeutung verlor.

Alle organischen Bindemittel besitzen jedoch den Nachteil, dass sie sich beim Abguss thermisch zersetzen und Schadstoffe, wie z.B. Benzol, Toluol oder Xylole, freisetzen können. Außerdem geben viele organische Bindemittelsysteme bereits bei der Kernherstellung und -lagerung Lösemittel an die Umwelt ab oder es werden unangenehm riechende Gase als Härtungskatalysatoren eingesetzt. Es ist zwar durch verschiedene Maßnahmen gelungen, alle diese Emissionen zu vermindern, vermeiden lassen sie sich bei organischen Bindemitteln jedoch nicht.

Aus diesem Grund gibt es seit einigen Jahren wieder vermehrt Bestrebungen, die anorganischen Bindemittel für die Kernherstellung sowie die zugehörigen Kernherstellungsverfahren weiterzuentwickeln.

AT341125 B offenbart eine Sandmischung für das Herstellen von Formen und Kernen nach dem Cold-Box oder Pep-Set-Verfahren zur Verwendung im Metallguss sowie ein Verfahren zum Aushärten jener Sandmischung, Als Bindemittel werden Polyurethane offenbart, welche zusammen mit Estern oder Anhydriden bestimmter Carbonsäuren wie Di- bzw. Tetrahydroabietinsäure, Linolsäure, Adipinsäure, Hydroxybenzoesäure, Maleinsäure und/oder Pimelinsäure eingesetzt werden.

Im Bereich der kalthärtenden Kernherstellungsverfahren beschreibt beispielsweise die DE 102012103705.1 eine Weiterentwicklung des Wasserglas-CO₂₋Verfahrens, indem die CO₂-Begasung mit einem zweiten Spülgas kombiniert wird. Zur Erzielung hoher Festigkeiten bei kurzen Taktzeiten wurde zudem der Weg verfolgt, die Aushärtung in einem heißen Werkzeug vorzunehmen und ggfs. zusätzlich heiße Luft durch die Formstoffmischung zu leiten, um das als Lösemittel vorhandene Wasser möglichst vollständig auszutreiben. Ein solches System wird z.B. in der EP 1802409 B1 (US 7770629) beschrieben. Diese heißaushärtenden Verfahren haben jedoch den Nachteil, dass die Werkzeuge beheizbar ausgeführt werden und die Aufheizung einen zusätzlichen Energieverbrauch verursacht, was eine erhebliche Kostenbelastung für das Verfahren darstellt.

### Aufgabe der Erfindung

Die Erfinder haben sich daher die Aufgabe gestellt, ein Verfahren zu entwickeln, das es erlaubt, Formen und Kerne unter Verwendung eines auf Wasserglas basierenden, anorganischen Bindemittels auch in unbeheizten Werkzeugen herzustellen, wobei die Festigkeiten, insbesondere unmittelbar nach der Entnahme aus dem Werkzeug, bei gleichem Binder und identischem Bindergehalt wesentlich höher sein sollen als bei den bisher hier bekannten Herstellungsverfahren, beispielsweise dem Wasserglas-CO₂-Verfahren.

### Zusammenfassung der Erfindung

Diese Aufgabe wird mit einem Verfahren mit den Merkmalen des Anspruchs 1 gelöst. Vorteilhafte Weiterentwicklungen des erfindungsgemäßen Verfahrens sind Gegenstand der abhängigen Patentansprüche oder nachfolgend beschrieben. Das Verfahren umfasst zumindest die folgenden Schritte
a) Bereitstellen einer Formstoffmischung zumindest umfassend einen feuerfesten Formgrundstoff und Wasserglas als Bindemittel in einem Formkasten,
b) Eintragen eines Gases in den Formkasten umfassend zumindest eine Carbonylverbindung mit einem Molekulargewicht von größer 43 g/mol und unterhalb 200 g/mol umfassend neben einer Carbonyl-Gruppe (C=O) zumindest ein weiteres Kohlenstoffatom zur Aushärtung der Formstoffmischung, wobei die Carbonylverbindung durch folgende Formel

   R¹R²C=O

   gekennzeichnet ist, worin
   R¹ für
      - ein Wasserstoff-Atom, oder
      - ein C1- oder C2-Alkyl, und
   R² für
      - eine über das Sauerstoffatom gebundene mit R substituierte Carboxyl-Gruppe R-C(=O)-O-
      steht mit R = jeweils unabhängig voneinander C1- bis C3-Kohlenwasserstoff, vorzugsweise C1- oder C2-Alkyl, und
   wobei der Siedepunkt der Carbonylverbindung größer 20°C und kleiner 200°C bei 1013 hPa ist
   Bevorzugt ist die Carbonylverbindung Essigsäureanhydrid und/oder HC(=O)-O-C(=O)-CH₃.

Überraschend wurde gefunden, dass Formen und Kerne aus einer Formstoffmischung, die ein anorganisches, auf Wasserglas basierendes Bindemittel enthält, nach dem wie folgt beschriebenen Verfahren unter Verwendung einer Carbonylverbindung hergestellt werden können. Die Festigkeiten, insbesondere unmittelbar nach Entnahme aus dem Kernkasten, sind für Kerne, die nach dem erfindungsgemäßen Verfahren hergestellt wurden, deutlich höher als für andere hier bekannte Verfahren wie beispielsweise die Aushärtung mit CO₂ und Luft.

Bei dem erfindungsgemäßen Verfahren wird zunächst eine Formstoffmischung aus mindestens einem feuerfesten Formgrundstoff und einem anorganischen, auf Wasserglas basierenden Bindemittel hergestellt, geformt und verdichtet. Im Anschluss daran wird eine wie nachfolgend beschriebene Carbonylverbindung mittels eines Trägergases wie z.B. Luft oder Stickstoff hindurchgeleitet.

Bei der Herstellung der Formstoffmischung wird i.A. so vorgegangen, dass der feuerfeste Formgrundstoff vorgelegt und das Bindemittel unter Rühren zugegeben wird. Es wird so lange weiter gerührt, bis eine gleichmäßige Verteilung des Bindemittels auf dem Formgrundstoff gewährleistet ist.

Die Formstoffmischung wird anschließend in die gewünschte Form gebracht. Dabei werden die für die Formgebung üblichen Verfahren angewendet. Beispielsweise kann die Formstoffmischung mittels einer Kernschießmaschine mit Hilfe von Druckluft in das Formwerkzeug geschossen werden. Die Härtung erfolgt anschließend, indem die erfindungsgemäße Carbonylverbindung mit Hilfe eines Trägergases durch das mit der Formstoffmischung gefüllte Werkzeug geleitet wird. Dazu wird die erfindungsgemäße Carbonylverbindung beispielsweise mit Hilfe einer Dosiervorrichtung, wie sie in modernen Begasungsgeräten integriert ist, in den Gasstrom des Trägergases eingebracht. Die Dosierung der erfindungsgemäßen Carbonylverbindung zum Trägergas erfolgt vorzugsweise am Anfang des Spülvorgangs. Eine weitere Möglichkeit ist es, die Carbonylverbindung gleichmäßig während des gesamten Spülvorgangs zum Trägergas hinzu zu dosieren.

### Detaillierte Beschreibung der Erfindung

Bevorzugte erfindungsgemäße Carbonylverbindungen der allgemeinen Formel

Ri-C(=O)-R₂

sind nachfolgend hinsichtlich R₁ und R₂ (unabhängig voneinander) mit Lewis-Strichformeln veranschaulicht.
R₁ = -H, -CH₃, -CH₂-CH₃,
R2 =

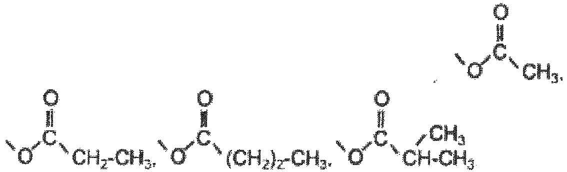

Insbesondere bevorzugt werden für R₁ Alkylgruppen mit mit einem Kohlenstoffatom ausgewählt.

Insbesondere bevorzugt werden für R₂ über das Sauerstoff-Atom gebundene Carboxyl-Gruppen mit mit 2 Kohlenstoffatomen ausgewählt.

Die erfindungsgemäße Carbonyl-Verbindung enthält zwei Carbonyl-Gruppen, die über ein Sauerstoff-Atom direkt miteinander verknüpft sind.

Bevorzugt ist die Carbonylverbindung Essigsäureanhydrid und/oder HC(=O)-O-C(=O)-CH₃.

Insbesondere war es für die Erfinder überraschend, dass der erfindungsgemäße Effekt ausschließlich mit den oben beschriebenen Carbonylverbindungen beobachtet werden kann, während sich andere Carbonylverbindungen als nicht geeignet herausgestellt haben.

Die erfindungsgemäße Carbonylverbindung weist bei Normaldruck (= 1013 hPa) einen Schmelzpunkt kleiner 100°C, bevorzugt kleiner 50°C und besonders bevorzugt kleiner 10°C auf.

Der Siedepunkt der erfindungsgemäßen Carbonylverbindung bei Normaldruck (=1013 hPa) liegt unterhalb 200°C, bevorzugt unterhalb 180°C und insbesondere bevorzugt unterhalb 160°C. In einer noch weiter bevorzugten Ausführungsform weist die erfindungsgemäße Carbonylverbindung bei Normaldruck (= 1013 hPa) einen Siedepunkt kleiner 143°C auf.

Die erfindungsgemäße Carbonylverbindung siedet bei Normaldruck (= 1013 hPa) oberhalb 20°C, bevorzugt oberhalb 30°C und insbesondere bevorzugt oberhalb 50°C. In einer noch weiter bevorzugten Ausführungsform liegt der Siedepunkt der erfindungsgemäßen Carbonylverbindung oberhalb 70°C.

Der Dampfdruck der erfindungsgemäßen Carbonylverbindung ist bei 20°C vorzugsweise größer als 3 mbar und besonders bevorzugt größer als 4 mbar. Gleichzeitig ist der Dampfdruck der erfindungsgemäßen Carbonylverbindung bei 20°C vorzugsweise kleiner 700 mbar, besonders bevorzugt kleiner 500 mbar und insbesondere bevorzugt kleiner 300 mbar. Die hier angegebenen Grenzen beziehen sich auf den Dampfdruck, wie er aus den Antoine-Konstanten berechnet werden kann und wie er in der Datenbank CHEMSAFE (Version 1.4.10 (2009)) der DECHEMA (Gesellschaft für Chemische Technik und Biotechnologie e.V.) veröffentlicht ist.

Die erfindungsgemäße Carbonylverbindung weist bei 20°C unter Normaldruck (= 1013 hPa) eine Löslichkeit in Wasser von bevorzugt größer 10 g/l, besonders bevorzugt von größer 50 g/l und insbesondere bevorzugt von größer 100 g/l auf. Die molare Masse der erfindungsgemäßen Carbonylverbindung liegt vorzugsweise unterhalb 200 g/mol, besonders bevorzugt unterhalb 150 g/mol und insbesondere bevorzugt unterhalb 120 g/mol. Des Weiteren hat die erfindungsgemäße Carbonyl-verbindung vorzugsweise eine molare Masse von größer 43 g/mol, besonders bevorzugt größer 60 g/mol und insbesondere bevorzugt von größer 80 g/mol.

Ohne an die Theorie gebunden zu sein, nehmen die Erfinder an, dass die erfindungsgemäße Carbonylverbindung als Aushärtungsreagenz dient. Es ist dabei überraschend, dass der erfindungsgemäße Effekt mit anderen Carbonylverbindungen nicht beobachtet werden kann.

In welcher Menge die erfindungsgemäße Carbonylverbindung eingesetzt wird, hängt insbesondere vom Gewicht des Kerns bzw. der auszuhärtenden Formstoffmischung ab. Bezogen auf die Formstoffmischung beträgt die benötigte Menge der erfindungsgemäßen Carbonylverbindung weniger als 5 Gew.% und besonders bevorzugt weniger als 1 Gew.%.

Die Untergrenze für die Zugabemenge der erfindungsgemäßen Carbonylverbindung beträgt vorzugsweise größer als 0,01 Gew.%, vorzugsweise größer als 0,03 Gew.%, besonders bevorzugt größer als 0,05 Gew.% und insbesondere bevorzugt größer als 0,1 Gew.%.

Nach einer anderen von der Formstoffmischung unabhängigen Definition wird die erfindungsgemäße Carbonylverbindung in einer Menge von vorzugsweise größer 1,25 Gew.-%, vorzugsweise größer 3,75 Gew.-%, besonders bevorzugt größer 6,25 Gew.-% und insbesondere bevorzugt größer 12,5 Gew.-% eingesetzt, bezogen auf die durch den Wasserglas-Binder zum Formstoff hinzugegebene Menge an Alkalisilikat, berechnet als die Summe aus Na₂O, K₂O, Li₂O und SiO₂. Gleichzeitig wird von der erfindungsgemäßen Carbonylverbindung, bezogen auf die Menge an Alkalisilikat, weniger als 625 Gew.-%, bevorzugt weniger als 250 Gew.-% und insbesondere bevorzugt weniger als 125 Gew.-% eingesetzt.

Unabhängig hiervon beträgt das molare Verhältnis des durch den Wasserglas-Binder zur Formstoffmischung hinzugegebenen, molaren Menge des Alkalimetalls M zur eingesetzten molaren Menge der erfindungsgemäßen Carbonylverbindung vorzugsweise 10:1 bis 1:1, insbesondere bevorzugt 8:1 bis 2:1 und ganz besonders bevorzugt 6:1 bis 3:1.

Die obigen drei Definitionen können auch gleichzeitig nebeneinander gelten.

Für das Trägergas, das zum Transport der erfindungsgemäßen Carbonylverbindung zur Formstoffmischung dient, sind aus wirtschaftlichen Gründen Luft und Stickstoff und insbesondere Luft bevorzugt. Die Begasungstemperatur des Trägergases liegt oberhalb von 10°C, vorzugsweise oberhalb von 20°C und besonders bevorzugt oberhalb von 35°C. Die Begasungstemperatur bezieht sich dabei wie auch in der weiteren Beschreibung des erfindungsgemäßen Verfahrens auf die Temperatur, die das Gas beim Eintritt in das Formwerkzeug aufweist. Eine Obergrenze besteht dabei nicht. Gegen die Verwendung sehr hoher Temperaturen sprechen in erster Linie wirtschaftliche Gründe, da die Preise der dafür notwendigen Erhitzer mit steigender Leistung stark ansteigen und der Aufwand für eine effektive Isolierung der Leitungen sehr groß ist.

Im Falle von Luft als Trägergas kann diese dem in den Gießereien üblicherweise vorhandenen Druckluftnetz entnommen werden, so dass der darin herrschende Druck aus rein praktischen Gründen die Obergrenze für die Begasung darstellt. Die Untergrenze, um einen effektiven Transport der Carbonylverbindung zur Formstoffmischung zu gewährleisten, liegt bei ca. 0,5 bar, vorzugsweise bei ca. 2 bar und besonders bevorzugt bei ca. 3 bar. Die Obergrenze ergibt sich aus der Druckbelastbarkeit des Formkastens und kann z.B. 10 bar betragen. Bei einem geringeren Druck würde sich die Begasungszeit stark verlängern, was mit einem Produktivitätsverlust verbunden wäre.

Wenn in dem vorliegenden Schutzrecht auf einen Formkasten Bezug genommen wird, meint dies, dass der Formkasten zur Herstellung von Formen und Kernen insbesondere Kernen, eingesetzt werden kann, und insofern einen Kernkasten umfasst.

Sämtliche Druckangaben, soweit nicht anders angegeben, beziehen sich jeweils auf Überdruck, d.h. einen Druck über dem Umgebungsdruck.

Die aus Polyurethan-Cold-Box-Verfahren bekannten Vorrichtungen, in welchem ein organisches Zweikomponentenbindersystem, ein Phenolharz und ein Polyisocyanat durch einen Katalysator, ein bei Raumtemperatur flüssiges Amin, ausgehärtet wird - der flüssige Katalysator wird zu diesem Zweck in die Dampf-/ Gasphase gebracht und mit Hilfe eines Trägergases durch die auszuhärtende Formstoffmischung geleitet - können auch für das vorliegende Verfahren eingesetzt werden. Eine allgemeine Beschreibung des Ashland-Polyurethan-ColdBox-Verfahrens findet sich im Gießerei-Lexikon nach Hasse et al. (19. Auflage, Schiele & Schöne Verlag 2008) unter dem Stichwort Cold-Box (Rubrik: PUR-Cold-Box: S.198+199). In der vorliegenden Erfindung übernimmt die Carbonylverbindung die Rolle des Aushärtungsreagenzes für das auf Wasserglas basierende Bindemittel. Bei Materialinkompatibilitäten mit einer Vorrichtung aus dem Polyurethan-Cold-Box-Verfahren werden die entsprechenden Vorrichtungsteile durch Materialien ersetzt, die gegenüber den erfindungsgemäßen Carbonylverbindungen beständig sind.

Die Carbonylverbindung liegt z.B. flüssig vor und wird durch ein Trägermedium in den Formkasten getragen, entweder als Aerosol oder bevorzugt gasförmig. Der Druckaufbau kann z.B. dadurch erfolgen, dass die Einblasöffnungen größer sind als die Ausblasöffnungen. Nicht verbrauchte Carbonylverbindung kann durch nachfolgendes Spülgas ausgetrieben werden.

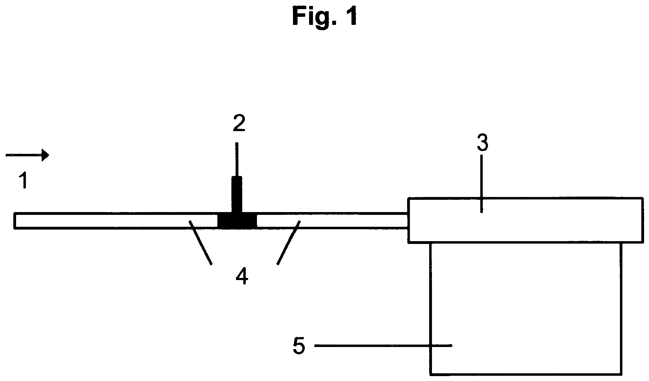

Beispielhaft ist eine geeignete Vorrichtung in Fig. 1 dargestellt. Durch die Luftzuleitung (4) wird Luft als Trägergas in Pfeilrichtung (1) durch eine Öffnung in der Begasungshaube (3) in den Formkasten (5) eingeblasen. Die Carbonylverbindung wird hierbei über das T-Stück (2) eingeführt und gasförmig mitgerissen.

Die Begasungszeit richtet sich nach dem Kerngewicht sowie der Kerngeometrie. Durch die geeignete Wahl der Begasungsparameter und des Layouts der Werkzeuge kann dafür gesorgt werden, dass auch bei größeren Kernen Herstellungszeiten möglich sind, die denen von organischen Bindemitteln entsprechen, z.B. weniger als 3 Minuten, bevorzugt weniger als 2,5 Minuten und besonders bevorzugt weniger als 2 Minuten. Eine solche Optimierung kann ggfs. auch mit Hilfe einer Computersimulation erfolgen.

Nach einer weiteren Ausführungsform kann dem Trägergas CO₂ beigemischt werden bzw. kann zusätzlich zum Trägergas CO₂ oder ein CO₂₋haltiges Gas verwendet werden, um die Festigkeiten der Kerne ggf. zu beeinflussen. Eine mögliche Ausgestaltung ist in der DE 102012103705.1 beschrieben.

Die Verwendung bereits vorhandener beheizbarer Formwerkzeuge ist bei dem beschriebenen Verfahren keineswegs ausgeschlossen, es eröffnet sich durch das erfindungsgemäße Verfahren die Möglichkeit, die Werkzeuge zur Kostensenkung entweder kalt, d.h. bei Umgebungs- bzw. Raumtemperatur von 15 bis 30°C, oder bei niedrigeren als den für das Heißhärtungsverfahren gewohnten Temperaturen zu betreiben, d.h. niedriger als 200°C, oder niedriger als 120°C oder sogar niedriger als 100°C, insbesondere können günstigere nicht-beheizbare Werkzeuge eingesetzt werden. Das erfindungsgemäße Verfahren schließt auch nicht aus, die Kerne bzw. Formen anschließend einer zusätzlichen Wärmebehandlung zu unterziehen.

Es ist ohne weiteres möglich, die erfindungsgemäße Härtung des Formstoffes durch bekannte Verfahren zu modifizieren, z.B. durch das Anlegen eines Vakuums. Außerdem lassen sich an den eigentlichen Härtungsvorgang weitere bekannte Schritte anschließen, beispielsweise eine Behandlung mit Mikrowellen oder eine Erwärmung im Ofen.

Als feuerfester Formgrundstoff können für die Herstellung von Gießformen übliche und bekannte Materialien verwendet werden. Geeignet sind beispielsweise Quarz-, Zirkon- oder Chromerzsand, Olivin, Vermiculit, Bauxit, Schamotte sowie künstliche Formgrundstoffe, insbesondere mit mehr als 50 Gew.% Quarzsand bezogen auf den feuerfesten Formgrundstoff. Dabei ist es nicht notwendig, ausschließlich Neusande einzusetzen. Im Sinne einer Ressourcenschonung und zur Vermeidung von Deponiekosten ist es sogar vorteilhaft, einen möglichst hohen Anteil an regeneriertem Altsand zu verwenden, wie er aus gebrauchten Formen durch Recyceln erhältlich ist.

Unter einem feuerfesten Formgrundstoff werden Stoffe verstanden, die einen hohen Schmelzpunkt (Schmelztemperatur) aufweisen. Vorzugsweise ist der Schmelzpunkt des feuerfesten Formgrundstoffs größer als 600°C, bevorzugt größer als 900°C, besonders bevorzugt größer als 1200°C und insbesondere bevorzugt größer als 1500°C.

Der feuerfeste Formgrundstoff macht vorzugsweise größer 80 Gew.%, insbesondere größer 90 Gew.%, besonders bevorzugt größer 95 Gew.% der Formstoffmischung aus.

Ein geeigneter Sand wird z.B. in der WO 2008/101668 A1 (= US 2010/173767 A1) beschrieben. Gleichfalls geeignet sind Regenerate einsetzbar, die durch Waschen und anschließende Trocknung zerkleinerter gebrauchter Formen erhältlich sind. Weniger bevorzugt sind Regenerate, die durch rein mechanische Behandlung gewonnenen wurden. In der Regel können die Regenerate mindestens ca. 70 Gew.% des feuerfesten Formgrundstoffs ausmachen, bevorzugt mindestens ca. 80 Gew.% und besonders bevorzugt größer 90 Gew.%.

Der mittlere Durchmesser der feuerfesten Formgrundstoffe liegt in der Regel zwischen 100 µm und 600 µm, bevorzugt zwischen 120 µm und 550 µm und besonders bevorzugt zwischen 150 µm und 500 µm. Die mittlere Partikelgröße lässt sich z.B. durch Siebung nach DIN 66165 (Teil 2) mit Analysensieben DIN ISO 3310-1 bestimmen. Besonders bevorzugt sind Teilchenformen mit größter Längenausdehnung zu kleinster Längenausdehnung (rechtwinkelig zueinander und jeweils für alle Raumrichtungen) von 1:1 bis 1:5 oder 1:1 bis 1:3, d.h. solche die z.B. nicht faserförmig sind.

Der feuerfeste Formgrundstoff weist vorzugsweise einen rieselfähigen Zustand auf, insbesondere um die erfindungsgemäße Formstoffmischung in üblichen Kernschießmaschinen verarbeiten zu können.

Das erfindungsgemäße Verfahren ist insbesondere für anorganische, auf Wasserglas basierende Bindemittel geeignet. Die Wassergläser enthalten gelöste Alkalisilikate und können durch Lösen von glasartigen Lithium-, Natrium- und Kaliumsilikaten in Wasser hergestellt werden. Das Wasserglas weist vorzugsweise ein molares Modul SiO₂/M₂O im Bereich von 1,6 bis 4,0, insbesondere 2,0 bis kleiner 3,5, auf, wobei M₂O für die Summe von Lithium-, Natrium- und Kalium-Oxid steht. Die Bindemittel können auch auf Wassergläsern basieren, die mehr als eins der genannten Alkaliionen enthalten, wie z.B. die aus DE 2652421 A1 (= GB1532847 A) bekannten lithiummodifizierten Wassergläser. Weiterhin können die Wassergläser auch mehrwertige Ionen enthalten wie z.B. die in EP 2305603 A1 (= WO 2011/042132 A1) beschriebenen Aluminium-modifizierten Wassergläser. Nach einer besonderen Ausführungsform wird ein Wasserglas mit einem definierten Anteil an Lithium-Ionen bzw. einem Verhältnis [Li₂O] / [M₂O] bzw. [Li₂Oₐₖₜᵢᵥ] / [M₂O] eingesetzt. [Li₂O] bzw. [Li₂Oₐₖₜᵢᵥ] errechnet sich dabei aus dem Anteil der im Wasserglas vorhandenen amorphen Lithiumsilikaten, Lithiumoxiden und Lithiumhydroxiden, wie es in der DE 102013106276 A1 beschrieben ist.

Die Wassergläser weisen einen Feststoffanteil im Bereich von 25 bis 65 Gew.% auf, vorzugsweise von 30 bis 55 Gew.%, insbesondere von 30 bis 50 Gew.%. Der Feststoffanteil bezieht sich auf die im Wasserglas enthaltene Menge an SiO₂ und M₂O.

Je nach Anwendung und gewünschtem Festigkeitsniveau werden zwischen 0,5 Gew.% und 5 Gew.% des auf Wasserglas basierenden Bindemittels eingesetzt, vorzugsweise zwischen 0,75 Gew.% und 4 Gew.%, besonders bevorzugt zwischen 1 Gew.% und 3,5 Gew.%, jeweils bezogen auf den Formgrundstoff. Die Angaben beziehen sich auf die Gesamtmenge des Wasserglasbinders, einschließlich des (insbesondere wässrigen) Lösungs- bzw. Verdünnungsmittels und des (etwaigen) Feststoffanteils (zusammen = 100 Gew.%).

Die erfindungsgemäße Formstoffmischung enthält in einer weiteren Ausführungsform einen Anteil eines partikulären amorphen Siliziumdioxids, um das Festigkeitsniveau der mit solchen Formstoffmischungen hergestellten Gießformen zu erhöhen. Synthetisch hergestelltes amorphes Siliciumdioxid ist hier besonders bevorzugt.

Die Teilchengröße des amorphen Siliziumdioxids beträgt vorzugsweise weniger als 300 µm, bevorzugt weniger als 200 µm, insbesondere bevorzugt weniger als 100 µm und weist z.B. eine mittlere Primärpartikelgröße zwischen 0,05 µm und 10 µm auf. Der Siebrückstand des partikulären amorphen SiO₂ bei einem Durchgang durch ein Sieb mit 125 µm Maschenweite (120 mesh) beträgt vorzugsweise nicht mehr als 10 Gew.%, besonders bevorzugt nicht mehr als 5 Gew.% und ganz besonders bevorzugt nicht mehr als 2 Gew.%. Unabhängig hiervon beträgt der Siebrückstand auf einem Sieb mit einer Maschenweite von 63 µm weniger als 10 Gew.-%, vorzugsweise weniger als 8 Gew.%. Die Bestimmung des Siebrückstands oder einer Teilchengrößenverteilung durch Sieben erfolgt dabei vorliegend nach dem in der DIN 66165 (Teil 2) beschriebenen Maschinensiebverfahren, wobei zusätzlich ein Kettenring als Siebhilfe verwendet wird.

Das nach der vorliegenden Erfindung vorzugsweise eingesetzte partikuläre amorphe Siliziumdioxid hat einen Wassergehalt von kleiner 15 Gew.%, insbesondere kleiner 5 Gew.% und besonders bevorzugt von kleiner 1 Gew.%. Das partikulare amorphe SiO₂ wird insbesondere als Pulver (einschließend Stäube) eingesetzt.

Als amorphes SiO₂ können sowohl synthetisch hergestellte als auch natürlich vorkommende Kieselsäuren eingesetzt werden. Letztere sind z.B. aus DE 102007045649 bekannt, sind aber nicht bevorzugt, da sie i.d.R. nicht unerhebliche kristalline Anteile enthalten und deshalb als karzinogen eingestuft sind.

Unter *synthetisch* wird nicht natürlich vorkommendes amorphes SiO₂ verstanden, d.h. dessen Herstellung eine bewusst durchgeführte chemische Reaktion umfasst, wie sie von einem Menschen veranlasst wird, z.B. die Herstellung von Kieselsolen durch lonenaustauschprozesse aus Alkalisilikatlösungen, die Ausfällung aus Alkalisilikatlösungen, die Flammhydrolyse von Siliciumtetrachlorid, die Reduktion von Quarzsand mit Koks im Lichtbogenofen bei der Herstellung von Ferrosilicium und Silicium. Das nach den beiden letztgenannten Verfahren hergestellte amorphe SiO₂ wird auch als pyrogenes SiO₂ bezeichnet.

Gelegentlich wird unter synthetischem amorphem Siliziumdioxid nur Fällungskieselsäure (CAS-Nr. 112926-00-8) und flammhydrolytisch hergestelltes SiO₂ (Pyrogenic Silica, Fumed Silica, CAS-Nr. 112945-52-5) verstanden, während das bei der Ferrosilicium- bzw. Siliciumherstellung entstandene Produkt lediglich als amorphes Siliziumdioxid (Silica Fume, Microsilica, CAS-Nr. 69012-64-12) bezeichnet wird. Für die Zwecke der vorliegenden Erfindung wird auch das bei der Ferrosilicium- bzw. Siliciumherstellung entstandene Produkt als amorphes SiO₂ verstanden.

Bevorzugt eingesetzt werden Fällungskieselsäuren und pyrogenes, d.h. flammhydrolytisch oder im Lichtbogen hergestelltes Siliziumdioxid. Insbesondere bevorzugt eingesetzt werden durch thermische Zersetzung von ZrSiO₄ hergestelltes amorphes Siliziumdioxid (beschrieben in der DE 102012020509) sowie durch Oxidation von metallischem Si mittels eines sauerstoffhaltigen Gases hergestelltes SiO₂ (beschrieben in der DE 102012020510). Bevorzugt ist auch Quarzglaspulver (hauptsächlich amorphes Siliziumdioxid), das durch Schmelzen und rasches Wiederabkühlen aus kristallinem Quarz hergestellt wurde, so dass die Partikel kugelförmig und nicht splittrig vorliegen (beschrieben in der DE 102012020511). Die mittlere Primärpartikelgröße des partikulären amorphen Siliziumdioxids kann zwischen 0,05 µm und 10 µm, insbesondere zwischen 0,1 µm und 5 µm, besonders bevorzugt zwischen 0,1 µm und 2 µm betragen. Die Primärpartikelgröße kann z.B. mit Hilfe von dynamischer Lichtstreuung (z.B. Horiba LA 950) bestimmt sowie durch Rasterelektronenmikroskop-Aufnahmen (REM-Aufnahmen mit z.B. Nova NanoSEM 230 der Firma FEI) überprüft werden. Des Weiteren konnten mit Hilfe der REM-Aufnahmen Details der Primärpartikelform bis in die Größenordnung von 0,01 µm sichtbar gemacht werden. Die Siliziumdioxid-Proben wurden für die REM-Messungen in destilliertem Wasser dispergiert und anschließend auf einem mit Kupferband beklebten Aluminiumhalter aufgebracht, bevor das Wasser verdampft wurde.

Des Weiteren wurde die spezifische Oberfläche des partikulären amorphen Siliziumdioxids mithilfe von Gasadsorptionsmessungen (BET-Verfahren) nach DIN 66131 bestimmt. Die spezifische Oberfläche des partikulärem amorphen SiO₂ liegt zwischen 1 und 200 m²/g, insbesondere zwischen 1 und 50 m²/g, besonders bevorzugt zwischen 1 und 30 m²/g. Ggfs. können die Produkte auch gemischt werden, z.B. um gezielt Mischungen mit bestimmten Partikelgrößenverteilungen zu erhalten.

Je nach Herstellungsart und Produzent kann die Reinheit des amorphen SiO₂ stark variieren. Als geeignet haben sich Typen mit einem Gehalt von mindestens 85 Gew.% Siliziumdioxid erwiesen, bevorzugt von mindestens 90 Gew.% und besonders bevorzugt von mindestens 95 Gew.%. Je nach Anwendung und gewünschtem Festigkeitsniveau werden zwischen 0,1 Gew.% und 2 Gew.% des partikulären amorphen SiO₂ eingesetzt, vorzugsweise zwischen 0,1 Gew.% und 1,8 Gew.%, besonders bevorzugt zwischen 0,1 Gew.% und 1,5 Gew.%, jeweils bezogen auf den Formgrundstoff.

Das Verhältnis von Wasserglasbinder zu partikulärem amorphen Siliziumdioxid kann innerhalb weiter Grenzen variiert werden. Dies bietet den Vorteil, die Anfangsfestigkeiten der Kerne, d.h. die Festigkeit unmittelbar nach der Entnahme aus dem Werkzeug, stark zu verbessern, ohne die Endfestigkeiten wesentlich zu beeinflussen. Dies ist vor allem im Leichtmetallguss von großem Interesse. Auf der einen Seite sind hohe Anfangsfestigkeiten erwünscht, um die Kerne nach ihrer Herstellung problemlos transportieren oder zu ganzen Kernpaketen zusammensetzen zu können, auf der anderen Seite sollten die Endfestigkeiten nicht zu hoch sein, um Schwierigkeiten beim Kernzerfall nach dem Abguss zu vermeiden, d.h. der Formgrundstoff sollte nach dem Gießen problemlos aus Hohlräumen der Gussform entfernt werden können.

Bezogen auf das Gesamtgewicht des Bindemittels Wasserglas (einschließlich Verdünnungs- bzw. Lösungsmittel) ist das amorphe SiO₂ in einem Anteil von 1 bis 80 Gew.%, vorzugsweise von 2 bis 60 Gew.%, enthalten, besonders bevorzugt von 3 bis 55 Gew.% und insbesondere bevorzugt zwischen 4 bis 50 Gew.%. Unabhängig hiervon ist eine Zugabemenge des amorphen SiO₂, bezogen auf das Verhältnis Feststoffanteil des Wasserglases (bezogen auf die Oxide, d.h. Gesamtmasse aus Alkalimetalloxid und Siliciumdioxid) zu amorphem SiO₂, von 10 : 1 bis 1 : 1,2 (Gewichtsteile) bevorzugt.

Die Zugabe des amorphen Siliziumdioxids kann gemäß EP 1802409 B1 sowohl vor als auch nach der Binderzugabe direkt zum Feuerfeststoff erfolgen, es kann aber auch, wie in EP 1884300 A1 (= US 2008/029240 A1) beschrieben, zuerst eine Vormischung des SiO₂ mit zumindest einem Teil des Binders oder Natronlauge hergestellt und diese dann dem Feuerfeststoff zugemischt werden. Der ggf. noch vorhandene, nicht für die Vormischung verwendete Binder bzw. Binderanteil kann dem Feuerfeststoff vor oder nach der Zugabe der Vormischung oder zusammen mit dieser zugegeben werden. Vorzugsweise wird das amorphe SiO₂ dem Feuerfeststoff vor der Binderzugabe zugegeben.

In einer weiteren Ausführungsform kann der Formstoffmischung Bariumsulfat zugesetzt sein, um die Oberfläche des Gussstücks, insbesondere aus Aluminium, weiter zu verbessern.

Das Bariumsulfat kann synthetisch hergestelltes als auch natürliches Bariumsulfat sein, d.h. in Form von Mineralien hinzugefügt sein, die Bariumsulfat enthalten, wie Schwerspat bzw. Baryt. Dieses wie auch andere Merkmale des geeigneten Bariumsulfats sowie der mit ihm hergestellten Formstoffmischung werden in der DE 102012104934 näher beschrieben und deren Offenbarungsgehalt wird insofern durch Bezugnahme auch zur Offenbarung des vorliegenden Schutzrechts gemacht. Das Bariumsulfat wird bevorzugt in einer Menge von 0,02 bis 5,0 Gew.%, besonders bevorzugt 0,05 bis 3,0 Gew.%, insbesondere bevorzugt 0,1 bis 2,0 Gew.% oder 0,3 bis 0,99 Gew.%, jeweils bezogen die gesamte Formstoffmischung, zugegeben.

In einer weiteren Ausführungsform können weiterhin zumindest Aluminiumoxide und/oder Aluminium/Silizium Mischoxide in partikulärer Form bzw. Metalloxide des Aluminiums und Zirkoniums in partikulärer Form in Konzentrationen zwischen 0,05 Gew.% und 4,0 Gew.%, vorzugsweise zwischen 0,1 Gew.% und 2,0 Gew.%, besonders bevorzugt zwischen 0,1 Gew.% und 1,5 Gew.% und insbesondere bevorzugt zwischen 0,2 Gew.% und 1,2 Gew.%, jeweils bezogen auf den Formgrundstoff, der erfindungsgemäßen Formstoffmischung zugegeben werden/sein, wie in der DE 102012113073 bzw. der DE 102012113074 näher beschrieben.

Insofern werden diese Schriften durch Referenzierung auch als Offenbarung für die vorliegende Erfindung geltend gemacht. Durch derartige Zusätze können nach dem Metallguss Gussstücke, insbesondere aus Eisen oder Stahl mit sehr hoher Oberflächenqualität erhalten werden, so dass nach der Entfernung der Gießform nur eine geringe oder sogar gar keine Nachbearbeitung der Oberfläche des Gussstücks erforderlich ist.

In einer weiteren Ausführungsform kann die erfindungsgemäße Formstoffmischung eine phosphorhaltige Verbindung umfassen. Dieser Zusatz ist bei sehr dünnwandigen Abschnitten einer Gießform bevorzugt. Es handelt sich dabei bevorzugt um anorganische Phosphorverbindungen, in denen der Phosphor bevorzugt in der Oxidationsstufe +5 vorliegt.

Die phosphorhaltige Verbindung liegt bevorzugt in Form eines Phosphats oder Phosphoroxids vor. Das Phosphat kann dabei als Alkali- bzw. als Erdalkalimetallphosphat vorliegen, wobei Alkalimetallphosphate und hierbei insbesondere die Natriumsalze besonders bevorzugt sind.

Als Phosphate können sowohl Orthophosphate als auch Polyphosphate, Pyrophosphate oder Metaphosphate eingesetzt werden. Die Phosphate können beispielsweise durch Neutralisation der entsprechenden Säuren mit einer entsprechenden Base, beispielsweise einer Alkalimetallbase, wie NaOH, oder ggf. auch einer Erdalkalimetallbase hergestellt werden, wobei nicht notwendigerweise alle negativen Ladungen des Phosphats durch Metallionen abgesättigt sein müssen.

Es können sowohl die Metallphosphate als auch die Metallhydrogenphosphate sowie die Metalldihydrogenphosphate eingesetzt werden, wie beispielsweise Na₃PO₄, Na₂HPO₄, und NaH₂PO₄. Ebenso können die wasserfreien Phosphate wie auch Hydrate der Phosphate eingesetzt werden. Die Phosphate können sowohl in kristalliner als auch in amorpher Form in die Formstoffmischung eingebracht sein.

Unter Polyphosphaten werden insbesondere lineare Phosphate verstanden, die mehr als ein Phosphoratom umfassen, wobei die Phosphoratome jeweils über Sauerstoffbrücken miteinander verbunden sind.

Polyphosphate werden durch Kondensation von Orthophosphationen unter Wasserabspaltung erhalten, sodass eine lineare Kette von PO₄-Tetraedern erhalten wird, die jeweils über Ecken verbunden sind. Polyphosphate weisen die allgemeine Formel (O(PO₃)ₙ)⁽ⁿ⁺²⁾⁻ auf, wobei n der Kettenlänge entspricht. Ein Polyphosphat kann bis zu mehreren hundert PO₄-Tetraedern umfassen. Bevorzugt werden jedoch Polyphosphate mit kürzeren Kettenlängen eingesetzt. Bevorzugt weist n Werte von 2 bis 100, insbesondere bevorzugt 5 bis 50 auf. Es können auch höher kondensierte Polyphosphate verwendet werden, d.h. Polyphosphate, in welchen die PO₄-Tetraeder über mehr als zwei Ecken miteinander verbunden sind und daher eine Polymerisation in zwei bzw. drei Dimensionen zeigen.

Unter Metaphosphaten werden zyklische Strukturen verstanden, die aus PO₄-Tetraedern aufgebaut sind, die jeweils über Ecken miteinander verbunden sind. Metaphosphate weisen die allgemeine Formel ((PO₃)n)ⁿ⁻ auf, wobei n mindestens 3 beträgt. Bevorzugt weist n Werte von 3 bis 10 auf.

Es können sowohl einzelne Phosphate verwendet werden als auch Gemische aus verschiedenen Phosphaten und/oder Phosphoroxiden.

Der bevorzugte Anteil der phosphorhaltigen Verbindung, bezogen auf den feuerfesten Formgrundstoff, beträgt zwischen 0,05 und 1,0 Gew.-%. Bevorzugt wird der Anteil der phosphorhaltigen Verbindung zwischen 0,1 und 0,5 Gew.% gewählt. Die phosphorhaltige, anorganische Verbindung enthält bevorzugt zwischen 40 und 90 Gew.%, insbesondere bevorzugt zwischen 50 und 80 Gew.% Phosphor, berechnet als P₂O₅. Die phosphorhaltige Verbindung kann an sich in fester oder gelöster Form der Formstoffmischung zugesetzt sein. Bevorzugt ist die phosphorhaltige Verbindung der Formstoffmischung als Feststoff zugesetzt.

Gemäß einer vorteilhaften Ausführungsform enthält die erfindungsgemäße Formstoffmischung einen Anteil an plättchenförmigen Schmiermitteln, insbesondere Grafit oder MoS₂. Die Menge des zugesetzten plättchenförmigen Schmiermittels, insbesondere Grafits, beträgt vorzugsweise 0,05 bis 1 Gew.%, besonders bevorzugt 0,05 bis 0,5 Gew.%, bezogen auf den Formgrundstoff.

Gemäß einer weiteren vorteilhaften Ausführungsform können auch oberflächenaktive Substanzen, insbesondere Tenside, eingesetzt werden, welche die Fließfähigkeit der Formstoffmischung verbessern. Geeignete Vertreter dieser Verbindungen sind z.B. in WO 2009/056320 (= US 2010/0326620 A1) beschrieben. Bevorzugt werden anionische Tenside für die erfindungsgemäße Formstoffmischung verwendet. Genannt seien hier insbesondere Tenside mit Schwefelsäure- oder Sulfonsäure-Gruppen. In der erfindungsgemäßen Formstoffmischung ist der reine oberflächenaktive Stoff, insbesondere das Tensid, bezogen auf das Gewicht des feuerfesten Formgrundstoffs bevorzugt in einem Anteil von 0,001 bis 1 Gew.-%, besonders bevorzugt 0,01 bis 0,2 Gew.-% enthalten.

Gemäß einer weiteren vorteilhaften Ausführungsform sind in der Formstoffmischung neben dem feuerfesten Formgrundstoff, dem Wasserglas als Bindemittel und dem partikulärem amorphen Siliziumdioxid auch ein oder mehrere pulverförmige oxidische Bor-Verbindungen enthalten. Als oxidische Bor-Verbindung sind geeignet: Borate, Borsäuren, Borsäureanhydride, Borosilikate, Borophosphate, Borophosphosilikate und deren Mischungen und insbesondere Borate wie Alkali- und/oder Erdalkaliborate, wobei die oxidische Bor-Verbindung bevorzugt keine organischen Gruppen enthält und die oxidische Bor-Verbindung aus B-O-B Strukturelementen aufgebaut ist.

Die oxidische Bor-Verbindung ist, bezogen auf den feuerfesten Formgrundstoff zu größer als 0,002 Gew.% und zu kleiner als 1,0 Gew.%, bevorzugt zu größer als 0,005 Gew.% und zu kleiner als 0,4 Gew.%, besonders bevorzugt zu größer als 0,01 Gew.% und zu kleiner als 0,2 Gew. % und insbesondere bevorzugt zu größer als 0,02 Gew.%. und zu kleiner als 0,1 Gew.% in der Formstoffmischung enthalten bzw. der Formstoffmischung zugesetzt.

In einer weiteren Ausführungsform können der erfindungsgemäßen Formstoffmischung mit der Additiv-Komponente organische Verbindungen, wie Kohlenhydrate, (gemäß EP 1802409B1 und WO2008/046651) zugesetzt sein. Ein geringer Zusatz von organischen Verbindungen kann für spezielle Anwendungen vorteilhaft sein - beispielsweise, um die thermische Ausdehnung der ausgehärteten Formstoffmischung zu regulieren. Allerdings ist ein solcher nicht bevorzugt, da dies wiederum mit Emissionen von CO₂ und anderen Pyrolyseprodukten verbunden ist.

Nach einer Ausführungsform der Erfindung enthält die Formstoffmischung organische Komponenten mit einem Anteil bis zu maximal 0,49 Gew.%, insbesondere bis zu maximal 0,19 Gew.% wobei die erfindungsgemäße Carbonylverbindung hier ausgenommen ist, d.h. die erfindungsgemäße Carbonylverbindungen wird nicht zu den Zugabemengen der organischen Komponenten hinzugezählt.

### Beispiele

### 1. Herstellung der Formstoffmischungen

Jeweils 100 Gewichtsteile (GT) Quarzsand H 32 der Quarzwerke Frechen GmbH wurden in die Schüssel eines Mischers der Fa. Hobart (Modell HSM 10) gegeben und nachfolgend 0,5 Gewichtsteile (GT) amorphes SiO₂ (Microsilica POS B-W 90 LD der Firma Possehl Erzkontor GmbH) zugegeben und eine Minute vermischt. Unter Rühren wurden anschließend 2 GT des Bindemittels zugegeben und eine Minute intensiv mit dem Sand vermischt. Das Bindemittel, das bereits am Vortag des Versuchs durch Mischen hergestellt wurde, bestand aus 90,9 GT Nuclesil 80 der BASF SE (molares Verhältnis [SiO₂] / [M₂O] ca. 2,32, Feststoffgehalt ca. 43,6%) und 9,1 GT vollentsalztem Wasser.

### 2. Herstellung der Prüfkörper

Für die Prüfung der Formstoffmischungen wurden quaderförmige Prüfriegel mit den Abmessungen 150 mm x 22,36 mm x 22,36 mm hergestellt (sog. Georg-Fischer-Riegel). Ein Teil der nach 1. hergestellten Formstoffmischungen wurde in die Vorratskammer einer Kernschießmaschine H 1 der Fa. Röperwerke AG überführt. Der Rest der Formstoffmischungen wurde bis zum Wiederauffüllen der Kernschießmaschine zum Schutz vor dem Austrocknen und gegen eine vorzeitige Reaktion mit dem in der Luft vorhandenen CO₂ in einem sorgfältig verschlossenen Gefäß aufbewahrt. Aus der Vorratskammer wurden die Formstoffmischungen mittels Druckluft (4 bar) in das Formwerkzeug eingebracht. Daran anschließend wurden die Prüfkerne wie folgt ausgehärtet:

### 2.1 Aushärtung mit Luft

Der Kernkasten wurde mit Luft für 60 Sekunden gespült, wobei die Temperatur ca. 25°C (Raumtemperatur) betrug. Des Weiteren wurden Kerne hergestellt, indem das Formwerkzeug mit Luft für 45 Sekunden gespült wurde. Die Temperatur der Luft, wie sie am Eintritt in das Formwerkzeug gemessen wurde, lag in diesem Fall bei 50°C. In beiden Fällen betrug der Druck der Luft 4 bar.

### 2.2 Aushärtung durch eine Kombination aus CO₂ und Luft

Bei der Kombination aus CO₂ und Luft wurde das Formwerkzeug zuerst für eine Sekunde mit CO₂ und im Anschluss daran für 59 Sekunden mit Luft gespült. Sowohl die Luft als auch das CO₂ wurden bei Raumtemperatur (ca. 25°C) und einem Druck von 4 bar verwendet.

### 2.3 Aushärtung durch Essigsäureanhydrid und Luft als Trägergas

Die Dosierung von Essigsäureanhydrid erfolgte mit Hilfe einer Laborspritze, die über eine volumetrische Skala verfügte. Nach Aufnahme der benötigten Menge an Essigsäureanhydrid wurde diese über ein T-Stück in die Luft-Zuleitung zum Formwerkzeug injiziert. Fig. 1 veranschaulicht die hier verwendete Versuchsanordnung. Der Abstand zwischen dem T-Stück und der Begasungshaube betrug ca. 1,5 m. Nach der Injektion des Essigsäureanhydrids wurde das Formwerkzeug über die Luft-Zuleitung für 60 Sekunden mit Luft gespült. Die verwendete Luft war bei Raumtemperatur (ca. 25°C) und hatte einen Druck von 4 bar. Die jeweilige Zugabemenge von Essigsäureanhydrid ist in Tabelle 2 zusammengefasst.

### 3. Festigkeitsuntersuchungen der hergestellten Prüfkörper

Zur Bestimmung der Biegefestigkeiten wurden die Prüfriegel aus dem Formwerkzeug entnommen und in ein Georg-Fischer-Festigkeitsprüfgerät, ausgerüstet mit einer 3-Punkt-Biegevorrichtung, eingelegt und die Kraft gemessen, welche zum Bruch der Prüfriegel führte. Die Biegefestigkeiten wurden sowohl unmittelbar, d.h. maximal 10 Sekunden nach der Entnahme (Sofortfestigkeiten) als auch ca. 24 Stunden nach der Herstellung (Endfestigkeiten) bestimmt.

Die Ergebnisse der Festigkeitsprüfungen sind in Tabelle 1 aufgeführt. Bei den hier angegebenen Werten handelt es sich um Mittelwerte aus Mehrfachbestimmungen an mindestens 4 Kernen.

### 4. Ergebnisse

Für die Beispiele 1.1 und 1.2 wurden die Prüfkörper ausschließlich mit Luft ausgehärtet, während bei den Beispielen 1.3 und 1.4 eine Kombination von CO₂ und Luft für die Aushärtung benutzt wurde. Die Beispiele 2.1 bis 2.10 sind für Prüfkörper, die mit dem erfindungsgemäßen Verfahren ausgehärtet wurden, wobei Luft als Trägergas verwendet wurde. Die jeweilige Begasungstemperatur, gemessen am Eintritt in das Formwerkzeug, und die Begasungszeit sind ebenfalls in Tabelle 1 bzw. 2 zusammengefasst.

**Tabelle 1:**

| Nicht erfindungsgemäße Beispiele; Versuchsbedingungen sowie Ergebnisse für die Aushärtung mit Luft bzw. durch eine Kombination von Luft und CO₂. Die Zugabemenge des Bindemittels (Zusammensetzung: 90,9 GT Nuclesil 80 der BASF SE (molares Verhältnis [SiO₂] / [M₂O] ca. 2,32, Feststoffgehalt ca. 43,6%) und 9,1 GT vollentsalztes Wasser) betrug in allen Beispielen 2 Gewichtsteile; die Zugabemenge des amorphen SiO₂ (Microsilica POS B-W 90 LD der Firma Possehl Erzkontor GmbH) betrug in allen Beispielen 0,5 Gewichtsteile. | | | | | |
|---|---|---|---|---|---|
| Beispiel-Nummer | Begasungszeit CO₂ [sec] | Begasungszeit Luft [sec] | Temperatur des CO₂ bzw. der Luft [°C] | Sofortfestigkeit [N/cm²] | Endfestigkeit [N/cm²] |
| Aushärtung mit Luft | | | | | |
| 1.1 | 0 | 60 | 25°C | 75 | 283 |
| 1.2 | 0 | 45 | 50°C | 93 | 173 |

| Aushärtung durch eine Kombination aus CO₂ und Luft | | | | | |
|---|---|---|---|---|---|
| 1.3 | 1 | 59 | 25°C | 90 | 243 |
| 1.4 | 1 | 44 | 50°C | 110 | 190 |

**Tabelle 2:**

| Erfindungsgemäße Beispiele; Versuchsbedingungen sowie Ergebnisse für die Aushärtung durch das erfindungsgemäße Verfahren, wobei Essigsäureanhydrid verwendet wurde. In allen Beispielen diente Luft als Trägergas. Die Zugabemenge des Bindemittels (Zusammensetzung: 90,9 GT Nuclesil 80 der BASF SE (molares Verhältnis [SiO₂] / [M₂O] ca. 2,32, Feststoffgehalt ca. 43,6%) und 9,1 GT vollentsalztes Wasser) betrug in allen Beispielen 2 Gewichtsteile; die Zugabemenge des amorphen SiO₂ (Microsilica POS B-W 90 LD der Firma Possehl Erzkontor GmbH) betrug in allen Beispielen 0,5 Gewichtsteile. | | | | | |
|---|---|---|---|---|---|
| Beispiel-Nummer | Begasungszeit Luft [sec] | Zugabemenge Essigsäureanhydrid [ml] | Temperatur der Luft [°C] | Sofortfestigkeit [N/cm²] | Endfestigkeit [N/cm²] |
| Aushärtung durch Essigsäureanhydrid und Luft als Trägergas | | | | | |
| 2.1 | 60 | 0,2 | 25°C | 95 | 280 |
| 2.2 | 60 | 0,4 | 25°C | 125 | 260 |
| 2.3 | 60 | 0,5 | 25°C | 130 | 290 |
| 2.4 | 60 | 1,0 | 25°C | 135 | 260 |
| 2.5 | 45 | 0,1 | 50°C | 103 | 180 |
| 2.6 | 45 | 0,3 | 50°C | 185 | 195 |
| 2.7 | 45 | 0,5 | 50°C | 220 | 225 |
| 2.8 | 45 | 0,6 | 50°C | 165 | 235 |
| 2.9 | 45 | 0,7 | 50°C | 175 | 205 |
| 2.10 | 45 | 1,0 | 50°C | 160 | 203 |

In Tabelle 1 sind zwei verschiedene nicht erfindungsgemäße Aushärtungsverfahren gegenüber gestellt: Die Aushärtung mit Luft sowie die Aushärtung durch eine Kombination aus CO₂ und Luft. Der Vergleich der Ergebnisse zeigt, dass durch das CO₂ die Sofortfestigkeiten erhöht werden können, während es auf die Endfestigkeiten keinen signifikanten Einfluss hat.

Die Tabelle 2 fasst die Ergebnisse für das erfindungsgemäße Verfahren zusammen. Im Vergleich zu den Beispielen 1.1 und 1.2 für die Aushärtung mit Luft weisen die Werte für die Sofortfestigkeiten der erfindungsgemäßen Beispiele 2.1 bis 2.10 deutlich höhere Werte auf. Dieser Effekt wird insbesondere bei einer Begasungstemperatur von 50°C deutlich. Die Sofortfestigkeiten können bei einer Begasungstemperatur von 25°C um bis zu 80% gesteigert werden, bei einer Begasungstemperatur von 50°C sind durch das erfindungsgemäße Verfahren sogar mehr als doppelt so hohe Sofortfestigkeiten möglich.

Vergleicht man die Werte der Beispiele 1.3 und 1.4 für eine Kombination aus CO₂ und Luft mit den erfindungsgemäßen Beispielen 2.1 bis 2.10, so ist ebenfalls eine deutliche Zunahme der Sofortfestigkeiten zu erkennen. So kann die Sofortfestigkeit bei einer Begasungstemperatur von 25°C bei den erfindungsgemäßen Beispielen um bis zu 50% gesteigert werden, bei einer Begasungstemperatur von 50°C ist sogar eine Verdoppelung der Sofortfestigkeit möglich.

Des Weiteren geht aus Tabelle 1 und 2 hervor, dass durch eine Erhöhung der Begasungstemperatur von 25 auf 50°C die Begasungszeit von 60 Sekunden auf 45 Sekunden verkürzt werden kann. Während die Endfestigkeiten bei der kürzeren Begasungszeit von 45 Sekunden und der Begasungstemperatur von 50°C niedriger sind, steigen die Sofortfestigkeiten sogar an.

Die Beispiele 2.1 bis 2.10 zeigen, dass die Sofortfestigkeiten stark von der verwendeten Menge an Essigsäureanhydrid abhängen. Der erfindungsgemäße Effekt ist insbesondere ab einer Zugabemenge von 0,3 ml deutlich zu erkennen. Bei einer Begasungstemperatur von 50°C ist hinsichtlich der Zugabemenge an Essigsäureanhydrid ein Optimum bei 0,5 ml zu erkennen. Für höhere Zugabemengen von Essigsäureanhydrid nehmen bei dieser Temperatur die Sofortfestigkeiten ab, obgleich die gemessenen Sofortfestigkeiten für eine Zugabemenge oberhalb von 0,5 ml immer noch höher sind als für die Aushärtung mit Luft oder durch eine Kombination aus CO₂ und Luft.

## Patentansprüche

1. Verfahren zur Herstellung von Formen oder Kernen umfassend zumindest die folgenden Schritte
a) Bereitstellen einer Formstoffmischung umfassend zumindest einen feuerfesten Formgrundstoff und Wasserglas als Bindemittel in einem Formkasten,
b) Eintragen eines Gases in den Formkasten umfassend zumindest eine Carbonylverbindung mit einem Molekulargewicht von größer 43 g/mol und unterhalb 200 g/mol umfassend neben einer Carbonyl-Gruppe (C=O) zumindest ein weiteres Kohlenstoffatom zur Aushärtung der Formstoffmischung,
wobei die Carbonylverbindung durch folgende Formel
R¹R²C=O
gekennzeichnet ist, worin
R¹ für
- ein Wasserstoff-Atom, oder
- ein C1- oder C2-Alkyl, und
R² für
- eine über das Sauerstoffatom gebundene mit R substituierte Carboxyl-Gruppe R-C(=O)-O-
steht mit R = jeweils unabhängig voneinander C1- bis C3-Kohlenwasserstoff, vorzugsweise C1- oder C2-Alkyl, und
wobei der Siedepunkt der Carbonylverbindung größer 20°C und kleiner 200°C bei 1013 hPa ist.

2. Das Verfahren nach Anspruch 1, wobei die Carbonylverbindung Essigsäureanhydrid und/oder HC(=O)-O-C(=O)-CH₃ ist.

3. Das Verfahren nach einem oder mehreren der vorhergehenden Ansprüche, wobei die Carbonylverbindung durch eines oder mehrere der folgenden Merkmale weiter gekennzeichnet ist:
- die Carbonylverbindung weist ein Molekulargewicht von größer 60 g/mol bis kleiner 150 g/mol und insbesondere bevorzugt von größer 80 g/mol bis kleiner 120 g/mol auf;
- die Carbonylverbindung weist einen Siedepunkt bei 1013 hPa von größer 30°C bis kleiner 180°C und besonders bevorzugt von größer 50°C bis kleiner 160°C auf;
- die Carbonylverbindung weist einen Dampfdruck bei 20°C von größer als 3 mbar, vorzugsweise von größer 4 mbar und besonders bevorzugt von größer 4 mbar bis kleiner 700 mbar auf.

4. Das Verfahren nach einem oder mehreren der vorhergehenden Ansprüche, wobei die Carbonylverbindung mittels eines Trägergas-Stromes in den Formkasten eingebracht wird und das Trägergas bzw. der Trägergas-Strom durch eines oder mehrere der folgenden Merkmale weiter gekennzeichnet ist:
- das Trägergas ist vorzugsweise ausgewählt aus der Gruppe Luft, Stickstoff, Kohlendioxid und deren Gemischen, vorzugsweise Luft oder Stickstoff, insbesondere mit einem Taupunkt von kleiner 10°C;
- der Trägergas-Strom weist eine Temperatur von größer 10°C auf, vorzugsweise größer 20°C und besonders bevorzugt von 35°C bis 120°C;
- das Trägergas wird mit einem Druck von größer 0,5 bar, vorzugsweise größer 2 bar, jeweils Überdruck, in den Formkasten eingebracht.

5. Das Verfahren nach einem oder mehreren der vorhergehenden Ansprüche, wobei der Formkasten nicht selbst, ggf. aber durch Eintrag eines Gasstroms beheizbar ist.

6. Das Verfahren nach einem oder mehreren der vorhergehenden Ansprüche, wobei die Formstoffmischung durch eines oder mehrere der folgenden Merkmale weiter gekennzeichnet ist:
- zu größer 80 Gew.%, vorzugsweise größer 90 Gew.% und besonders bevorzugt zu größer 95 Gew.%, ist der feuerfeste Formgrundstoff, bezogen auf die Formstoffmischung, in der Formstoffmischung enthalten;
- zwischen 0,5 Gew.% und 5 Gew.%, vorzugsweise zwischen 1 Gew.% und 3,5 Gew.%, ist Wasserglas, jeweils einschließlich des Wassers und relativ zum Formgrundstoff in der Formstoffmischung enthalten;
- zwischen 0,1 Gew.% und 3,5 Gew.%, vorzugsweise zwischen 0,15 Gew.% und 2,5 Gew.%, ist Wasserglas, jeweils bezogen auf den Feststoffgehalt an Alkalisilikaten definiert als M₂O und SiO₂ des eingesetzten Wasserglases und relativ zur Formstoffmischung, enthalten, wobei M₂O für die Summe von Lithium-, Natrium- und Kalium-Oxid steht.

7. Das Verfahren nach einem oder mehreren der vorhergehenden Ansprüche, wobei die Formstoffmischung durch eines oder mehrere der folgenden Merkmale weiter gekennzeichnet ist:
- zu größer 0,01 bis kleiner 5 Gew,%, vorzugsweise 0,05 bis kleiner 5 Gew,%, besonders bevorzugt 0,1 bis kleiner 1 Gew,% ist die Carbonylverbindung, bezogen auf die Formstoffmischung, in der Formstoffmischung enthalten bzw. eingetragen;
- zu größer 1,25 Gew.% bis weniger als 625 Gew.%, vorzugsweise größer 3,75 Gew.% bis weniger als 250 Gew.%, und besonders bevorzugt größer 6,25 Gew.-% und weniger als 125 Gew.%, wird die Carbonylverbindung, bezogen auf die durch den Wasserglas-Binder zum Formstoff hinzugegebene Menge an Alkalisilikat und berechnet als Summe aus Na₂O, K₂O, Li₂O und SiO₂, eingesetzt,
- Wasserglas-Binder, berechnet als molare Menge der Alkalimetalle M, und Carbonylverbindung werden in einem molaren Verhältnis von 10:1 bis 1:1, vorzugsweise 8:1 bis 2:1 und besonders bevorzugt 6:1 bis 3:1 eingesetzt.

8. Das Verfahren nach einem oder mehreren der vorhergehenden Ansprüche, wobei der feuerfeste Formgrundstoff durch eines oder mehrere der folgenden Merkmale weiter gekennzeichnet ist:
- der feuerfeste Formgrundstoff umfasst zu mehr als 50 Gew.% Quarzsand bezogen auf den feuerfesten Formgrundstoff;
- der feuerfeste Formgrundstoff weist eine Schmelztemperatur von größer 600°C, bevorzugt größer als 1200°C, auf;
- der feuerfeste Formgrundstoff weist einen mittleren Partikeldurchmesser von 100 µm bis 600 µm, bestimmt nach DIN 66165 (Teil 2) mit Analysensieben nach DIN ISO 3310-1, auf;
- der feuerfeste Formgrundstoff umfasst Quarz-, Zirkon- oder Chromerzsand, Olivin, Vermiculit, Bauxit, Schamotte, Glasperlen, Glasgranulat, Aluminiumsilikatmikrohohlkugeln und deren Mischungen und besteht vorzugsweise zu mehr als 50 Gew.% aus Quarzsand, bezogen auf den feuerfesten Formgrundstoff.

9. Das Verfahren nach einem oder mehreren der vorhergehenden Ansprüche, wobei die Formstoffmischung partikuläres amorphes Siliziumdioxid enthält und das partikuläre amorphe Siliziumdioxid vorzugsweise als eine Komponente separat vom Wasserglas und separat vom feuerfesten Formgrundstoff der Formstoffmischung zugegeben wird.

10. Das Verfahren nach Anspruch 9, wobei die Formstoffmischung durch eines oder mehrere der folgenden Merkmale weiter gekennzeichnet ist:
• zu 0,1 bis 2 Gew.%, vorzugsweise zu 0,1 bis 1,5 Gew.%, ist partikuläres amorphes Siliziumdioxid, relativ zum feuerfesten Formgrundstoff in der Formstoffmischung enthalten;
• zu 2 bis 60 Gew.%, besonders bevorzugt zu 4 bis 50 Gew.% ist partikuläres amorphes Siliziumdioxid, relativ zum Gewicht des Wasserglases ,einschließlich Wasser, in der Formstoffmischung enthalten, wobei der Feststoffanteil des Wasserglases 25 bis 65 Gew.%, vorzugsweise von 30 bis 55 Gew.%, beträgt;
• zu 4 bis 50 Gew.% ist partikuläres amorphes Siliziumdioxid, relativ zum Gewicht des Wasserglases in der Formstoffmischung enthalten, wobei das Gewicht des Wasserglases jeweils bezogen auf den Feststoffgehalt an Alkalisilikaten in Form von M₂O und SiO₂ definiert ist, wobei M₂O für die Summe von Lithium-, Natrium- und Kalium-Oxid steht.

11. Das Verfahren nach Anspruch 9 oder 10, wobei das partikuläre amorphe Siliziumdioxid durch eines oder mehrere der folgenden Merkmale weiter gekennzeichnet ist:
- das partikuläre amorphe Siliziumdioxid weist eine nach BET bestimmte Oberfläche zwischen 1 und 200 m²/g, vorzugsweise größer gleich 1 m²/g und kleiner gleich 30 m²/g, besonders bevorzugt von kleiner gleich 15 m²/g. auf;
- das partikuläre amorphe Siliziumdioxid weist einen mittleren durch dynamische Lichtstreuung bestimmten primären Partikeldurchmesser zwischen 0,05 µm und 10 µm, insbesondere zwischen 0,1 µm und 5 µm und besonders bevorzugt zwischen 0,1 µm und 2 µm, auf;
- das partikuläre amorphe Siliziumdioxid ist aus der Gruppe bestehend aus: Fällungskieselsäure, flammhydrolytisch oder im Lichtbogen hergestellten pyrogenen Siliziumdioxid, durch thermische Zersetzung von ZrSiO₄ hergestelltem amorphen Siliziumdioxid, durch Oxidation von metallischem Silizium mittels eines sauerstoffhaltigen Gases hergestelltem Siliziumdioxid, Quarzglaspulver mit kugelförmigen Partikeln, das durch Schmelzen und rasches Wiederabkühlen aus kristallinem Quarz hergestellt wurde, und deren Mischungen ausgewählt;
- das eingesetzte partikuläre amorphe Siliziumdioxid weist einen Wassergehalt von kleiner 15 Gew.%, vorzugsweise kleiner 5 Gew.%, und besonders bevorzugt kleiner 1 Gew.% auf.

12. Das Verfahren nach einem oder mehreren der vorhergehenden Ansprüche, wobei das Wasserglas ein molares Modul SiO₂/M₂O im Bereich von 1,6 bis 4,0, insbesondere 2,0 bis kleiner 3,5, aufweist, wobei M₂O für die Summe aus Lithium-, Natrium- und Kalium-Oxid steht.

13. Das Verfahren nach einem oder mehreren der vorhergehenden Ansprüche, wobei die Formstoffmischung nachfolgend dem Aussetzen mit der Carbonyl-Verbindung einem Gasstrom von einer Temperatur von 100 bis 300 °C, vorzugsweise von 120 bis 250 °C ausgesetzt wird, vorzugsweise für unter 5 min.

14. Formen oder Kerne für den Metallguss, insbesondere den Aluminium-Guss, herstellbar nach einem Verfahren nach zumindest einem der vorhergehenden Ansprüche.

15. Eine Formstoffmischung zur Herstellung von Formen oder Kernen umfassend zumindest:
- einen feuerfesten Formgrundstoff;
- Wasserglas als Bindemittel;
- partikuläres amorphes Siliziumdioxid; und
- zumindest eine Carbonylverbindung, wobei die Carbonylverbindung durch folgende Formel
R¹R²C=O
gekennzeichnet ist, worin
R¹ für
- ein Wasserstoff-Atom, oder
- ein C1- oder C2-Alkyl,
R² für
- eine über das Sauerstoffatom gebundene substituierte Carboxyl-Gruppe R-C(=O)-O-
steht mit R = jeweils unabhängig voneinander C1- bis C3-Kohlenwasserstoff, vorzugsweise C1- oder C2-Alkyl, und
wobei der Siedepunkt der Carbonylverbindung größer 20°C und kleiner 200°C bei 1013 hPa ist.

16. Ein Mehrkomponenten-System zur Herstellung von Formen oder Kernen umfassend zumindest die folgenden räumlich separat voneinander vorliegenden Komponenten (B) und (G):
(B) eine flüssige Binder-Komponente (B) umfassend zumindest
- Wasserglas enthaltend Wasser, und
(G) zumindest eine Carbonylverbindung, wobei die Carbonylverbindung durch folgende Formel
R¹R²C=O
gekennzeichnet ist, worin
R¹ für
- ein Wasserstoff-Atom, oder
- ein C1-oder C2-Alkyl,
R² für
- eine über das Sauerstoffatom gebundene substituierte Carboxyl-Gruppe R-C(=O)-O-
steht mit R = jeweils unabhängig voneinander C1- bis C3-Kohlenwasserstoff, vorzugsweise C1- oder C2-Alkyl, und
wobei der Siedepunkt der Carbonylverbindung größer 20°C und kleiner 200°C bei 1013 hPa ist.

17. Das Mehrkomponenten-System nach Anspruch 16 zur Herstellung von Formen oder Kernen umfassend weiterhin die folgende räumlich separat voneinander vorliegende Komponente (F),
(F) zumindest eine rieselfähige Feuerfest-Komponente (F) umfassend
- einen feuerfesten Formgrundstoff und
- kein Wasserglas.

18. Die Mehrkomponenten-System nach Anspruch 16 oder 17 zur Herstellung von Formen oder Kernen umfassend weiterhin die folgende räumlich ebenfalls separat von den anderen Komponenten vorliegende Komponente (A),
(A) eine pulverförmige Additiv-Komponente umfassend zumindest
- partikuläres amorphes Siliciumdioxid und
- kein Wasserglas.

19. Die Formstoffmischung nach Anspruch 15 oder das Mehrkomponenten-System nach einem oder mehreren der Ansprüche 16 bis 18, wobei die Carbonylverbindung Essigsäureanhydrid und/oder HC(=O)-O-C(=O)-CH₃ ist/sind.

## Claims

1. A method of making moulds or cores, comprising at least the following steps:
a) providing a moulding material mixture which comprises at least one refractory moulding base material and waterglass as a binder in a moulding box;
b) introducing a gas into the moulding box, the gas comprising at least one carbonyl compound having a molecular weight of greater than 43 g/mol and less than 200 g/mol and comprising, in addition to a carbonyl group (C=O), at least one further carbon atom for curing the moulding material mixture;
wherein the carbonyl compound is **characterised by** the following formula:
R¹R²C=O
wherein
R¹ is
- a hydrogen atom, or
- a C1 or C2 alkyl, and
R² is
- an R-C(=O)-O- carboxyl group that is substituted by R and bonded via the oxygen atom,
where R = C1 to C3 hydrocarbon, preferably C1 or C2 alkyl, each independently of each other, and
wherein the boiling point of the carbonyl compound is greater than 20 °C and less than 200 °C at 1013 hPa.

2. The method according to claim 1, wherein the carbonyl compound is acetic anhydride and/or HC(=O)-O-C(=O)-CH₃.

3. The method according to one or more of the preceding claims, wherein the carbonyl compound is further **characterised by** one or more of the following features:
- the carbonyl compound has a molecular weight of greater than 60 g/mol to less than 150 g/mol and more preferably of greater than 80 g/mol to less than 120 g/mol;
- the carbonyl compound has a boiling point at 1013 hPa of greater than 30 °C to less than 180 °C and more preferably of greater than 50 °C to less than 160 °C;
- the carbonyl compound has a vapour pressure at 20 °C of greater than 3 mbar, preferably of greater than 4 mbar and most preferably of greater than 4 mbar to less than 700 mbar.

4. The method according to one or more of the preceding claims, wherein the carbonyl compound is introduced into the moulding box by means of a carrier gas flow, and the carrier gas or carrier gas flow is further **characterised by** one or more of the following features:
- the carrier gas is preferably selected from the group consisting of air, nitrogen, carbon dioxide and mixtures thereof, preferably air or nitrogen, in particular with a dew point of less than 10 °C;
- the carrier gas flow has a temperature of greater than 10 °C, preferably greater than 20 °C and more preferably from 35 °C to 120 °C;
- the carrier gas is introduced into the moulding box at a pressure of greater than 0.5 bar, preferably greater than 2 bar, this being an excess pressure in each case.

5. The method according to one or more of the preceding claims, wherein the moulding box is not itself heatable, but where appropriate heatable by introducing a gas flow.

6. The method according to one or more of the preceding claims, wherein the moulding material mixture is further **characterised by** one or more of the following features:
- greater than 80% by weight, preferably greater than 90% by weight and most preferably greater than 95% by weight of the refractory moulding base material is contained in the moulding material mixture, based on the moulding material mixture;
- between 0.5% by weight and 5% by weight, preferably between 1 % by weight and 3.5% by weight of waterglass is contained in the moulding material mixture, in each case including the water and relative to the moulding base material in the moulding material mixture;
- between 0.1% by weight and 3.5% by weight, preferably between 0.15% by weight and 2.5% by weight of waterglass is contained, in each case based on the solids content of alkali silicates defined as M2O and SiO₂ of the waterglass used and relative to the moulding material mixture, wherein M2O is the sum of lithium oxide, sodium oxide and potassium oxide.

7. The method according to one or more of the preceding claims, wherein the moulding material mixture is further **characterised by** one or more of the following features:
- greater than 0.01% to less than 5% by weight, preferably 0.05% to less than 5% by weight, most preferably 0.1% to less than 1% by weight of the carbonyl compound is contained in or introduced into the moulding material mixture, based on the moulding material mixture;
- greater than 1.25% by weight to less than 625% by weight, preferably greater than 3.75% by weight to less than 250% by weight, and most preferably greater than 6.25% by weight and less than 125% by weight of the carbonyl compound is used, based on the amount of alkali silicate that is added to the moulding material by the waterglass binder and calculated as the sum of Na₂O, K₂O, Li₂O and SiO₂;
- the waterglass binder, calculated as the molar amount of the alkali metals M, and the carbonyl compound are used in a molar ratio of 10:1 to 1:1, preferably 8:1 to 2:1 and most preferably 6:1 to 3:1.

8. The method according to one or more of the preceding claims, wherein the refractory moulding base material is further **characterised by** one or more of the following features:
- the refractory moulding base material comprises more than 50% by weight of silica sand based on the refractory mould base material;
- the refractory moulding base material has a melting temperature of greater than 600 °C, preferably greater than 1200 °C;
- the refractory moulding base material has an average particle diameter of 100 µm to 600 µm, determined in accordance with DIN 66165 (Part 2) using test sieves in accordance with DIN ISO 3310-1;
- the refractory moulding base material comprises silica sand, zircon sand or chromium ore sand, olivine, vermiculite, bauxite, fireclay, glass beads, glass granulate, hollow microspheres of aluminium silicate, and mixtures thereof and preferably consists of more than 50% by weight of silica sand, based on the refractory moulding base material.

9. The method according to one or more of the preceding claims, wherein the moulding material mixture comprises particulate amorphous silicon dioxide and the particulate amorphous silicon dioxide is preferably added to the moulding compound as a component separate from the waterglass and separate from the refractory moulding base material of the moulding material mixture.

10. The method according to claim 9, wherein the moulding material mixture is further **characterised by** one or more of the following features:
• from 0.1% to 2% by weight, preferably from 0.1% to 1.5% by weight, of particulate amorphous silicon dioxide is contained in the moulding material mixture, relative to the refractory moulding base material;
• from 2% to 60% by weight, more preferably from 4% to 50% by weight, of particulate amorphous silicon dioxide is contained in the moulding material mixture, relative to the weight of the waterglass including water, wherein the solids content of the waterglass is 25% to 65% by weight, preferably 30% to 55% by weight;
• from 4% to 50% by weight of particulate amorphous silicon dioxide is contained in the moulding material mixture, relative to the weight of the waterglass, wherein the weight of the waterglass is in each case defined based on the solids content of alkali silicates in the form of M₂O and SiO₂, wherein M₂O is the sum of lithium oxide, sodium oxide and potassium oxide.

11. The method according to claim 9 or 10, wherein the particulate amorphous silicon dioxide is further **characterised by** one or more of the following features:
- the particulate amorphous silicon dioxide has a surface area of between 1 and 200 m²/g, preferably greater than or equal to 1 m²/g and less than or equal to 30 m²/g, most preferably less than or equal to 15 m²/g, the surface area being determined according to BET;
- the particulate amorphous silicon dioxide has an average primary particle diameter of between 0.05 µm and 10 µm, more particularly between 0.1 µm and 5 µm and most preferably between 0.1 µm and 2 µm, the particle diameter being determined by dynamic light scattering;
- the particulate amorphous silicon dioxide is selected from the group consisting of: precipitated silicon dioxide, pyrogenic silicon dioxide produced by flame hydrolysis or in the electric arc, amorphous silicon dioxide produced by thermal decomposition of ZrSiO₄, silicon dioxide produced by oxidation of metallic silicon by means of an oxygen-containing gas, spherical-particle silica powder produced by melting and rapid re-cooling of crystalline quartz, and mixtures thereof;
- the particulate amorphous silicon dioxide used has a water content of less than 15% by weight, preferably less than 5% by weight, and most preferably less than 1% by weight.

12. The method according to one or more of the preceding claims, wherein the waterglass has an SiO₂/M₂O molar modulus in the range of 1.6 to 4.0, in particular 2.0 to less than 3.5, wherein M₂O is the sum of lithium oxide, sodium oxide and potassium oxide.

13. The method according to one or more of the preceding claims, wherein the moulding material mixture is subsequently exposed to a gas flow having a temperature of 100 to 300 °C, preferably of 120 to 250 °C, preferably for less than 5 min, after exposure to the carbonyl compound.

14. Moulds or cores for metal casting, in particular aluminium casting, producible by a method according to at least one of the preceding claims.

15. A moulding material mixture for making moulds or cores, at least comprising:
- a refractory moulding base material;
- waterglass as a binder;
- particulate amorphous silicon dioxide; and
- at least one carbonyl compound, wherein the carbonyl compound is **characterised by** the following formula:
R¹R²C=O
wherein
R¹ is
- a hydrogen atom, or
- a C1 or C2 alkyl, and
R² is
- an R-C(=O)-O- carboxyl group that is substituted and bonded via the oxygen atom,
where R = C1 to C3 hydrocarbon, preferably C1 or C2 alkyl, each independently of each other, and
wherein the boiling point of the carbonyl compound is greater than 20 °C and less than 200 °C at 1013 hPa.

16. A multi-component system for producing moulds or cores, comprising at least the following spatially separate components (B) and (G):
(B) a liquid binder component (B), at least comprising
- waterglass containing water, and
(G) at least one carbonyl compound, wherein the carbonyl compound is **characterised by** the following formula:
R¹R²C=O
wherein
R¹ is
- a hydrogen atom, or
- a C1 or C2 alkyl, and
R² is
- an R-C(=O)-O- carboxyl group that is substituted and bonded via the oxygen atom,
where R = C1 to C3 hydrocarbon, preferably C1 or C2 alkyl, each independently of each other, and
wherein the boiling point of the carbonyl compound is greater than 20 °C and less than 200 °C at 1013 hPa.

17. The multi-component system according to claim 16 for making moulds or cores, further comprising the following spatially separate component (F),
(F) at least one free-flowing refractory component (F) comprising
- a refractory moulding base material, and
- no waterglass.

18. The multi-component system according to claim 16 or 17 for making moulds or cores, further comprising the following component (A) also spatially separate from the other components,
(A) a powdery additive component, at least comprising
- particulate amorphous silicon dioxide, and
- no waterglass.

19. The moulding material mixture according to claim 15 or the multi-component system according to one or more of claims 16 to 18, wherein the carbonyl compound is/are acetic anhydride and/or HC(=O)-O-C(=O)-CH₃.

## Revendications

1. Procédé de fabrication de moules ou de noyaux comprenant au moins les étapes suivantes consistant à :
a) préparer un mélange de matières de moulage comprenant au moins une matière de base de moulage réfractaire et du verre soluble comme liant dans un châssis de moule,
b) introduire un gaz dans le châssis de moule comprenant au moins un composé carbonyle ayant un poids moléculaire supérieur à 43 g/mole et inférieur à 200 g/mole comprenant, en plus d'un groupe carbonyle (C=O), au moins un autre atome de carbone pour le durcissement du mélange de matières de moulage,
dans lequel le composé carbonyle est **caractérisé par** la formule suivante :
R¹R²C=O
dans laquelle
R¹ représente
- un atome d'hydrogène, ou
- un alkyle en C₁ ou C₂, et
R₂ représente
- un groupe carboxyle R-C(=O)-O- substitué par R, lié par le biais de l'atome d'oxygène,
avec R = hydrocarbure en C₁ à C₃ respectivement indépendamment l'un de l'autre, de préférence groupe alkyle en C₁ ou C₂, et
dans lequel le point d'ébullition du composé carbonyle est supérieur à 20°C et inférieur à 200°C à 1013 hPa.

2. Procédé selon la revendication 1, dans lequel le composé carbonyle est un acide acétique anhydre et/ou HC(=O)-O-C(=O)-CH₃.

3. Procédé selon l'une ou plusieurs des revendications précédentes, dans lequel le composé carbonyle est en outre **caractérisé par** l'une ou plusieurs des caractéristiques suivantes :
- le composé carbonyle présente un poids moléculaire supérieur à 60 g/mol et inférieur à 150 g/mol et de manière particulièrement préférée, supérieur à 80 g/mol et inférieur à 120 g/mol ;
- le composé carbonyle présente un point d'ébullition à 1013 hPa supérieur à 30°C et inférieur à 180°C et de manière particulièrement préférée, supérieur à 50°C et inférieur à 160°C ;
- le composé carbonyle présente une pression de vapeur à 20°C supérieure à 3 mbar, de préférence supérieure à 4 mbar et de manière particulièrement préférée supérieure à 4 mbar et inférieure à 700 mbar.

4. Procédé selon l'une ou plusieurs des revendications précédentes, dans lequel le composé carbonyle est introduit dans le châssis de moule au moyen d'un courant de gaz support et le gaz support et/ou le courant de gaz support est en outre **caractérisé par** l'une ou plusieurs des caractéristiques suivantes :
- le gaz support est de préférence choisi dans le groupe air, azote, dioxyde de carbone et leurs mélanges, de préférence air ou azote, ayant en particulier un point de rosée inférieur à 10°C ;
- le courant de gaz support présente une température supérieure à 10°C, de préférence supérieure à 20°C et de manière particulièrement préférée de 35°C à 120°C ;
- le gaz support est introduit dans le châssis de moule à une pression supérieure à 0,5 bar, de préférence supérieure à 2 bar, respectivement sous surpression.

5. Procédé selon l'une ou plusieurs des revendications précédentes, dans lequel le châssis de moule n'est pas auto-chauffant, mais le cas échéant chauffant par apport d'un gaz support.

6. Procédé selon l'une ou plusieurs des revendications précédentes, dans lequel le mélange de matières de moulage est en outre **caractérisé par** l'une ou plusieurs des caractéristiques suivantes :
- la matière de base de moulage réfractaire est présente dans le mélange de matières de moulage à plus de 80 % en poids, de préférence à plus de 90 % en poids, et de manière particulièrement préférée à plus de 95 % en poids, par rapport au mélange de matières de moulage ;
- le verre soluble est compris dans le mélange de matières de moulage entre 0,5 % en poids et 5 % en poids, de préférence entre 1 % en poids et 3,5 % en poids, respectivement en incluant l'eau et par rapport à la matière de base de moulage ;
- le verre soluble est compris entre 0,1 % en poids et 3,5 % en poids, de préférence entre 0,15 % en poids et 2,5 % en poids, respectivement par rapport à la teneur en silicates alcalins solides définis en tant que M₂O et SiO₂ du verre soluble mis en œuvre et par rapport au mélange de matières de moulage, M₂O représentant la somme des oxydes de lithium, sodium et potassium.

7. Procédé selon l'une ou plusieurs des revendications précédentes, dans lequel le mélange de matières de moulage est en outre **caractérisé par** l'une ou plusieurs des caractéristiques suivantes :
- le composé carbonyle est compris et/ou introduit dans le mélange de matières de moulage à plus de 0,01 jusqu'à moins de 5 % en poids, de préférence à plus de 0,05 jusqu'à moins de 5 % en poids, de manière particulièrement préférée à plus de 0,1 jusqu'à moins de 1 % en poids, par rapport au mélange de matières de moulage ;
- le composé carbonyle est mis en œuvre à plus de 1,25 % en poids jusqu'à moins de 625 % en poids, de préférence à plus de 3,75 % en poids jusqu'à moins de 250 % en poids, et de manière particulièrement préférée à plus de 6,25 % en poids et jusqu'à moins de 125 % en poids, par rapport à la quantité de silicate alcalin ajoutée par le liant de verre soluble à le matière de moulage et calculée en tant que somme de Na₂O, K₂O, Li₂O et SiO₂,
- le liant de verre soluble, calculé sous forme de quantité molaire des métaux alcalins M, et le composé carbonyle sont mis en œuvre en un rapport molaire de 10 : 1 à 1 : 1, de préférence 8 : 1 à 2 : 1, et de manière particulièrement préférée de 6:1 à 3:1.

8. Procédé selon l'une ou plusieurs des revendications précédentes, dans lequel la matière de base de moulage réfractaire est en outre **caractérisée par** l'une ou plusieurs des caractéristiques suivantes :
- la matière de base de moulage réfractaire comprend à plus de 50 % en poids du sable de quartz par rapport à la matière de base de moulage réfractaire ;
- la matière de base de moulage réfractaire présente une température de fusion supérieure à 600°C, de préférence supérieure à 1200°C ;
- la matière de base de moulage réfractaire présente un diamètre moyen de particules de 100 µm à 600 µm, déterminée d'après la norme DIN 66165 (partie 2) avec des tamis d'analyse selon la norme DIN ISO 3310-1 ;
- la matière de base de moulage réfractaire comprend du sable de quartz, de zircone ou de minerai de chrome, de l'olivine, de la vermiculite, de la bauxite, de la chamotte, des perles de verre, du granulé de verre, des microsphères creuses de silicate d'aluminium et leurs mélanges et se compose de préférence à plus de 50 % en poids de sable de quartz, par rapport à la matière de base de moulage réfractaire.

9. Procédé selon l'une ou plusieurs des revendications précédentes, dans lequel le mélange de matières de moulage contient du dioxyde de silicium particulaire amorphe et le dioxyde de silicium particulaire amorphe est ajouté de préférence en tant que composé séparément du verre soluble et séparément de la matière de base de moulage réfractaire du mélange de matières de moulage.

10. Procédé selon la revendication 9, dans lequel le mélange de matières de moulage est en outre **caractérisé par** l'une ou plusieurs des caractéristiques suivantes :
• le dioxyde de silicium particulaire amorphe est compris à 0,1 jusqu'à 2 % en poids, de préférence à 0,1 jusqu'à 1,5 % en poids, par rapport à la matière de base de moulage réfractaire dans le mélange de matières de moulage ;
• le dioxyde de silicium particulaire amorphe est présent dans le mélange de matières de moulage à 2 jusqu'à 60 % en poids, de manière particulièrement préférée à 4 jusqu'à 50 % en poids, par rapport au poids du verre soluble, y compris l'eau, la proportion de matières solides du verre soluble étant de 25 à 65 % en poids, de préférence de 30 à 55 % en poids ;
• le dioxyde de silicium amorphe particulaire est compris dans le mélange de matières de moulage à 4 jusqu'à 50 % en poids, par rapport au poids du verre soluble, le poids du verre soluble étant défini respectivement par rapport à la teneur en silicates alcalins solides sous forme de M₂O et SiO₂, M₂O représentant la somme des oxydes de lithium, de sodium, et de potassium.

11. Procédé selon la revendication 9 ou 10, dans lequel le dioxyde de silicium particulaire amorphe est en outre **caractérisé par** l'une ou plusieurs des caractéristiques suivantes :
- le dioxyde de silicium particulaire amorphe présente une surface déterminée d'après BET comprise entre 1 et 200 m²/g, de préférence supérieure ou égale à 1 m²/g et inférieure ou égale à 30 m²/g, de manière particulièrement préférée inférieure ou égale à 15 m²/g ;
- le dioxyde de silicium particulaire amorphe présente un diamètre moyen de particules primaires déterminé par dispersion de la lumière dynamique compris entre 0,05 µm et 10 µm, en particulier entre 0,1 µm et 5 µm et de manière particulièrement préférée entre 0,1 µm et 2 µm :
- le dioxyde de silicium particulaire amorphe est choisi dans le groupe constitué de : acide silicique de précipitation, dioxyde de silicium pyrogène fabriqué par hydrolyse à la flamme ou à arc, dioxyde de silicium amorphe fabriqué par décomposition thermique de ZrSiO₄, dioxyde de silicium fabriqué par oxydation de silicium métallique au moyen d'un gaz contenant de l'oxygène, poudre de verre de quartz comportant des particules sphériques, qui a été fabriquée par fusion et refroidissement rapide à partir de quartz cristallin, et leurs mélanges ;
- le dioxyde de silicium particulaire amorphe présente une teneur en eau inférieure à 15 % en poids, de préférence inférieure à 5 % en poids et de manière particulièrement préférée inférieure à 1 % en poids.

12. Procédé selon l'une ou plusieurs des revendications précédentes, dans lequel le verre soluble présente un module molaire SiO₂/M₂O dans la plage de 1,6 à 4,0, en particulier de 2,0 à moins de 3,5, M₂O représentant la somme des oxydes de lithium, de sodium, et de potassium.

13. Procédé selon l'une ou plusieurs des revendications précédentes, dans lequel le mélange de matières de moulage, à la suite de la soumission au composé carbonyle, est soumis à un courant de gaz ayant une température de 100 à 300°C, de préférence de 120 à 250°C, de préférence pendant moins de 5 min.

14. Moules ou noyaux pour la coulée de métaux, en particulier la coulée d'aluminium, pouvant être fabriqués d'après un procédé selon au moins l'une des revendications précédentes.

15. Mélange de matières de moulage pour la fabrication de moules ou de noyaux comprenant au moins :
- une matière de base de moulage réfractaire ;
- du verre soluble comme liant ;
- du dioxyde de silicium particulaire amorphe ; et
- au moins un composé carbonyle, le composé carbonyle étant **caractérisé par** la formule suivante :
R¹R²C=O
dans laquelle
R¹ représente
- un atome d'hydrogène, ou
- un alkyle en C₁ ou C₂, et
R₂ représente
- un groupe carboxyle R-C(=O)-O- substitué, lié par le biais de l'atome d'oxygène,
avec R = hydrocarbure en C₁ à C₃ respectivement indépendamment l'un de l'autre, de préférence groupe alkyle en C₁ ou C₂, et
dans lequel le point d'ébullition du composé carbonyle est supérieur à 20°C et inférieur à 200°C à 1013 hPa.

16. Système multicomposant pour la fabrication de moules ou de noyaux comprenant au moins les composants (B) et (G) suivants présents séparément physiquement l'un de l'autre :
(B) un composant de liant liquide (B) comprenant au moins
- de l'eau contenant du verre soluble, et
(G) au moins un composé carbonyle, le composé carbonyle étant **caractérisé par** la formule suivante :
R¹R²C=O
dans laquelle
R¹ représente
- un atome d'hydrogène, ou
- un alkyle en C₁ ou C₂, et
R₂ représente
- un groupe carboxyle R-C(=O)-O- substitué, lié par le biais de l'atome d'oxygène,
avec R = hydrocarbure en C₁ à C₃ respectivement indépendamment l'un de l'autre, de préférence groupe alkyle en C₁ ou C₂, et
dans lequel le point d'ébullition du composé carbonyle est supérieur à 20°C et inférieur à 200°C à 1013 hPa.

17. Système multicomposant selon la revendication 16 pour la fabrication de moules ou de noyaux comprenant en outre le composant (F) suivant présent séparément physiquement des autres :
(F) au moins un composant réfractaire écoulable (F) comprenant
- une matière de base de moulage réfractaire et
- aucun verre soluble.

18. Système multicomposant selon la revendication 16 ou 17 pour la fabrication de moules ou de noyaux comprenant en outre le composant (A) suivant présent également séparément physiquement des autres composants,
(A) un composant additif pulvérulent comprenant au moins
- du dioxyde de silicium particulaire amorphe et
- aucun verre soluble.

19. Mélange de matières de moulage selon la revendication 15 ou système multicomposant selon l'une ou plusieurs des revendications 16 à 18, dans lequel le composé carbonyle est un acide acétique anhydre et/ou HC(=O)-O-C(=O)-CH₃.
